# EUROPEAN PATENT SPECIFICATION

(11) **EP 3 871 602 B1**
(45) Date of publication and mention of the grant of the patent: **27.09.2023**
(21) Application number: 20159511.3
(22) Date of filing: 26.02.2020
(51) Int. Cl.: A61B 5/145, A61B 5/00, A61L 2/28

(54) **MEDICAL SYSTEM AND METHOD OF STERILITY TESTING THE MEDICAL SYSTEM**
MEDIZINISCHES SYSTEM UND VERFAHREN ZUR STERILITÄTSPRÜFUNG DES MEDIZINISCHEN SYSTEMS
SYSTÈME MÉDICAL ET PROCÉDÉ DE TEST DE STÉRILITÉ DU SYSTÈME MÉDICAL

(43) Date of publication of application: 01.09.2021
(73) Proprietor: Roche Diabetes Care GmbH, 68305 Mannheim (DE); F. Hoffmann-La Roche AG, 4070 Basel (CH)
(72) Inventor: List, Hans, 68305 Mannheim (DE); Riebel, Stefan, 68305 Mannheim (DE); Rittinghaus, Andrea, 68305 Mannheim (DE)
(74) Representative: Altmann Stößel Dick Patentanwälte PartG mbB

(56) References cited:
- WO-A1-2019/122095
- US-A- 4 351 900
- US-A1- 2012 328 473

## Description

### Technical Field

The invention relates to a medical system and to methods of providing the medical system and of sterility testing of the medical system. The medical system may be used for detecting at least one analyte in a body fluid, such as a body fluid contained in a body tissue. The medical system may specifically be configured for the method of sterility testing. The medical system may be applied in the field of continuous monitoring of at least one analyte in a body fluid of a user, specifically applied in the field of home care and in the field of professional care, such as in hospitals. Other applications are feasible.

### Background art

Monitoring certain body functions, more particularly monitoring one or more concentrations of at least one analyte concentration such as at least one metabolite concentration in a body fluid plays an important role in the prevention and treatment of various diseases. Such analytes can include by way of example, but not exclusively, glucose, lactate, cholesterol or other types of analytes and metabolites. Without restricting further possible applications, the invention will be described in the following text with reference to glucose monitoring. However, additionally or alternatively, the invention can also be applied to other types of analytes, such as the analytes mentioned above.

Generally, medical systems for long-term monitoring of an analyte in a body tissue of a user as well as corresponding insertion devices are known. As an example, WO 2019/122095 A1 discloses a medical system, comprising a housing and a preassembled functional module received in the housing. The pre-assembled functional module comprises an analytical sensor for detecting at least one analyte in a body fluid of a user, an electronics unit electrically connected to the analytical sensor and an insertion component for inserting the analytical sensor into a body tissue of the user. The medical system further comprises at least one removable protective cap connected to the housing, covering the preassembled functional module.

US4351900A discloses a method and apparatus for testing the sterility of the dry contents of a sterile ampoule. A dual channel needle is inserted into the container and a dissolving solution is injected into the ampoule to form a solution. A rinsing solution is circulated into the interior of the apparatus to remove that solution which is then directed to means for culturing and incubating a filtrate of the solution to determine whether microorganisms are present therein. The needle is retained by hand into the ampoule and held by the cap of the closed transfer chamber during rinsing.

Despite the advantages achieved by the above-mentioned devices, several technical challenges remain. Specifically, medical products which may be delivered sterile to the customer may either be sterilized completely inside their packaging unit or the sterile compounds may be enclosed to the product kit in a separate sterile sealing. The user may have to assemble these devices prior to application.

In case no product kit shall be delivered to the customer but instead a fully integrated device comprising sterile parts and other parts which may be damaged by sterilization, the sterile parts may be further processed after the sterilization without any contamination. During the assembly of the device possible contamination might occur. Therefore, the complete product may have to be tested whether the sterile parts were contaminated or not, at least on a random basis. However, in these fully integrated devices, the sterile parts are generally not accessible anymore. A removal of the sterile parts may require opening of the sterile sealing and, thus, may cause a possible contamination.

### Problem to be solved

It is therefore desirable to provide devices and methods which at least partially address the above-mentioned technical challenges. Specifically, a medical system is desirable which allows for easy and user-friendly insertion of a an analyte sensor into a body tissue and for continuous monitoring of an analyte in the body tissue of a user, while enabling the manufacturer to verify the sterility of sterile parts of these devices.

### Summary

This problem is addressed by a medical system and a method of sterility testing thereof with the features of the independent claims. Preferred embodiments of the present invention are defined in the dependent claims.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1a-b: show an embodiment of a preassembled functional module in a cross-sectional view (Figure 1a) and in a perspective view (Figure 1b);
- Figure 2: shows a first embodiment of a medical system in a cross-sectional view;
- Figure 3a-d: show a second embodiment of a medical system in cross-sectional views (Figures 3a and 3c) and in perspective views (Figures 3b and 3d), with a removable stopper (Figures 3a and 3b) and with a fluidic adapter (Figures 3c and 3d);
- Figure 4: shows an enlarged view of a sterility cap of the medical system of Figures 3a and 3b in a cross-sectional view;
- Figure 5: shows an enlarged view of the sterility cap of the medical system of Figures 3c and 3d in a cross-sectional view;
- Figure 6: shows a flow chart of an embodiment of a method of sterility testing of a medical system;
- Figure 7: shows a flow chart of an embodiment of a method of providing a medical system; and
- Figures 8a-b: show a cross-sectional view (Figure 8a) and a perspective view (Figure 8b) of a subgroup forming a part of the preassembled functional module of Figures 1a and 1b, ready for sterilization and before assembly with the remaining parts of the functional module.

### Detailed description of the embodiments

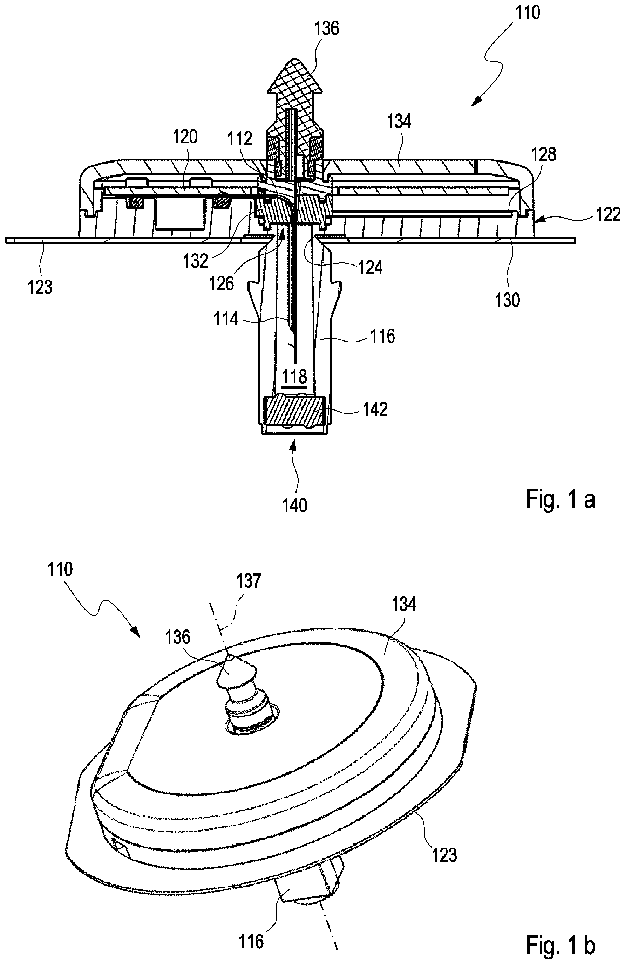

In Figures 1a-b, an exemplary embodiment of a preassembled functional module 110 is shown in a cross-sectional view (Figure 1a) and in a perspective view (Figure 1b). The preassembled functional module 110 comprises at least one analyte sensor 112 for detecting at least one analyte in a body fluid of a user. The analyte sensor 112 may be or may comprise an electrochemical sensor and may be configured for continuous monitoring of the analyte in the body fluid of the user. The analyte sensor 112 may be inserted into a body tissue of the user by using an insertion component 114 of the preassembled functional module 110, for example an insertion cannula or an insertion needle.

The insertion component 114 is at least partially surrounded by at least one sterility cap 116. The sterility cap 116 surrounds the insertion component 114 and at least a part of the analyte sensor 112, such as a distal part or implantable part thereof. The sterility cap 116 may provide an interior space 118 which specifically may be sterilized and sealed from the outside. The insertion component 114 and at least part of the analyte sensor 112 may be located within the interior space 118.

The analyte sensor 112 is electrically connected to an electronics unit 120 of the preassembled functional module 110. The electronics unit 120 may be configured for performing at least one electronic function, specifically for performing an analyte measurement, such as by measuring at least one of an electrical current and/or a voltage provided by the analyte sensor 112. Thus, the electronics unit 120 may be configured for receiving an electronic signal, such as an electrical current and/or a voltage, from the analyte sensor 112 and further, optionally, for determining the analyte concentration in the body fluid of the user by using the electrical current and/or the voltage. The preassembled functional module 110 may further comprise at least one electrical energy storage device, for example a battery, which specifically may be configured for supplying electrical energy to the electronics unit 120.

Further, the preassembled functional module 110 may comprise at least one base plate 122. The base plate 122, as an example, may be designed as a body mount and may comprise an adhesive surface, such as an adhesive plaster 123, for mounting the base plate 122 to a skin site of the user.

The sterility cap 116 may be connected to the base plate 122. For example, the base plate 122 and the sterility cap 116 may be manufactured as one single piece, such as by a common molding process. Alternatively, the sterility cap 116 may be connected to the base plate 122 by means of at least one of a welding technique; a bonding technique; a soldering technique; an adhesive method. The connection between the sterility cap 116 and the base plate 122 may form a predetermined breaking point 124, such as a circumferential breaking line, as will be outlined in further detail below.

The base plate 122 may be configured for receiving the electronics unit 120 and at least part of the analyte sensor 112, such as a part configured for being inserted into the body tissue of the user. The analyte sensor 112 and the insertion component 114 may penetrate the base plate 122 via at least one through hole 126 from an upper side 128 to a lower side 130 of the base plate 122. On the lower side 130, the sterility cap 116 may surround the through hole 126. On the upper side 128, the analyte sensor 112 may be mounted to the base plate 122, such as by at least one sensor fixation element 132, whereby the sensor fixation element 132 may seal the through hole 126.

The preassembled functional module 110 may further comprise at least one cover element 134. The cover element 134 may cover the electronics unit 120 and at least part of the analyte sensor 112. The cover element 134 may be disposed on the base plate 122 on the upper side 128.

Further, the preassembled functional module 110 may comprise at least one plunger 136 which may be part of the insertion component 114 and/or which may be connected to the insertion component 114. The plunger 136 may be movable with respect to the base plate 122 and/or with respect to a housing 146 of the medical system 144 which will be described in further detail below. The base plate 122 may be applied to the skin site of the user when the analyte sensor 112 is inserted into the body tissue of the user, such as by the adhesive plaster 123. Thus, the base plate 122 may comprise the adhesive plaster 123 for fixing the preassembled functional module 110 to the skin site of the user.

The preassembled functional module 110 may have a rotational symmetry, for example an axial rotational symmetry about an axis 137 such as a cylinder axis, as can be seen in Figure 1b showing the preassembled functional module 110 in a perspective view. Even though the symmetry may simplify assembly, other geometries are possible as well.

In another embodiment, the preassembled functional module 110 does not have a rotational symmetry. This may allow easier manufacturing of the medical system 144 as the exact assembling of the preassembled functional module 110 with the housing 146 is easier.

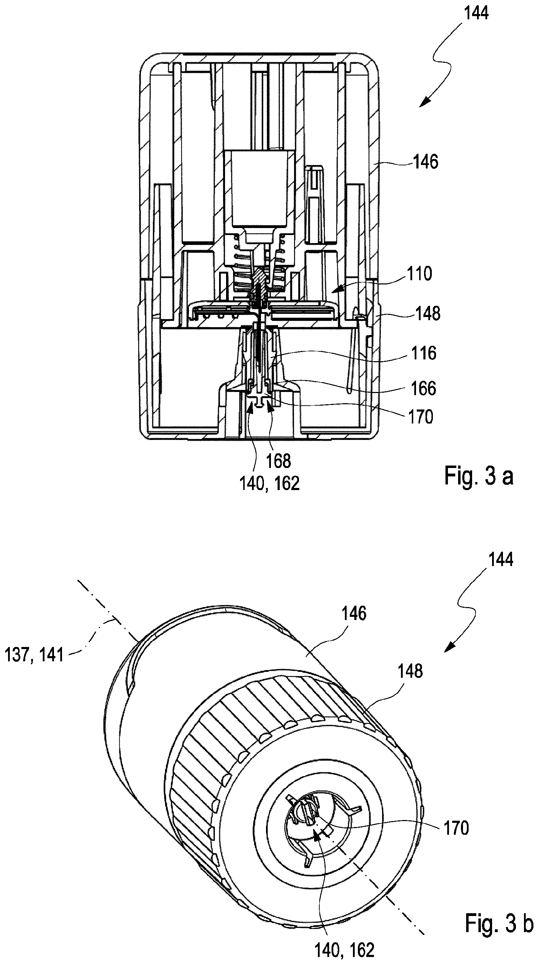

The sterility cap 116 of the preassembled functional module 110 further comprises at least one sterility testing access 140, as will be described in further detail below. The sterility testing access 140 comprises at least one of a septum 142 and a multiple-step sealing 162. In the embodiment shown in Figures 1a-b, the sterility testing access 140 comprises the at least one septum 142. In other embodiments, such as shown in Figures 3a-d, the sterility testing access 140 may comprise the at least one multiple-step sealing 162, as will be explained in further detail below.

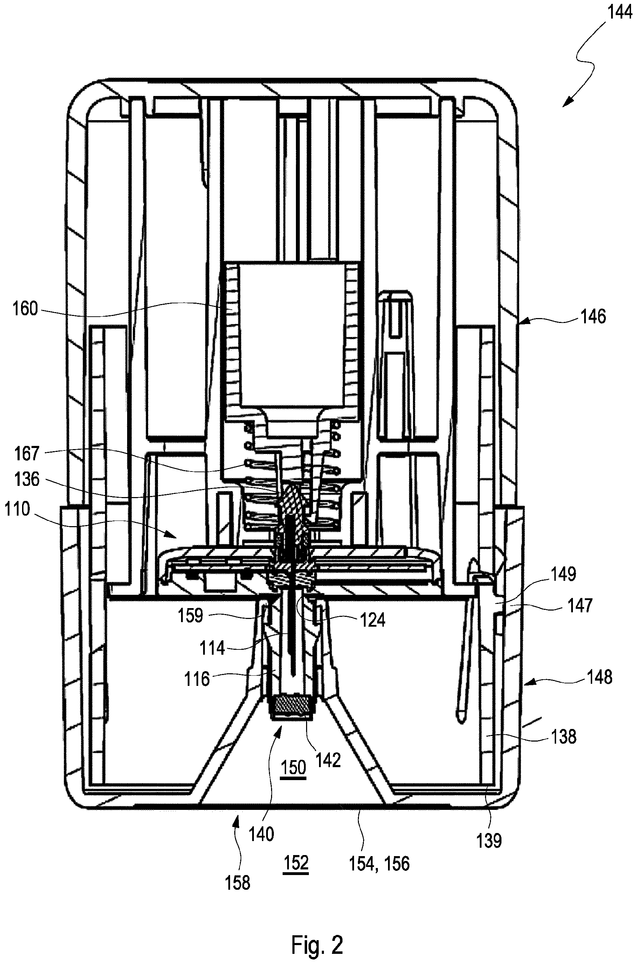

In Figure 2, a first exemplary embodiment of a medical system 144 is shown in a cross-sectional view. The medical system 144 comprises a housing 146 which, for example, may be made of a plastic material and/or a metallic material. The housing 146 may be configured for providing protection for the enclosed parts, such as against mechanical and/or environmental influences, e.g. humidity.

The medical system 144 comprises the preassembled functional module 110, wherein the preassembled functional module 110 is received in the housing 146. The medical system 144 may comprise the embodiment of the preassembled functional module 110 shown in Figures 1a-b. For the description of the preassembled functional module 110, reference is therefore made to the description of Figures 1a-b.

The medical system 144 further comprises at least one removable protective cap 148. The removable protective cap 148 may fully or partially be made of a plastic material and/or a metallic material. The removable protective cap 148 may be connected to the housing 146 by a form-fit or a force-fit connection.

For example, a rim 147 of the removable protective cap 148 may be pushed over a rim 149 of the housing 146 or vice a versa. As another example, the removable protective cap 148 may be connected to the housing 146 via a threaded joint. The rims 147, 149 may form a tight connection or sealing. The removable protective cap 148 may be removed by the user by at least one of pulling the removable protective cap 148 off the housing 146; turning the removable protective cap 148 off the housing 146.

Further, the removable protective cap 148 may comprise a funnel-shaped depression 150. Thus, the sterility cap 116, specifically the sterility testing access 140 of the sterility cap 116, may be accessible from an outer side 152 of the medical system 144. The sterility testing access 140 may specifically be accessible through the removable protective cap 148, more specifically through the removable protective cap 148 when the removable protective cap 148 is connected to the housing 146. In Figure 2, the sterility testing access 140 exemplarily comprises the septum 142.

An access to the sterility cap 116 may be sealed by a removable seal 154, for example by a removable liner 156. The removable liner 156 specifically may cover an opening 158 in the removable protective cap 148 through which the sterility cap 116 may be accessible after removing the removable seal 154. The opening 158 specifically may be an opening of the funnel-shaped depression 150.

Further, as can be seen in Figure 2, the removable protective cap 148 may surround the sterility cap 116 within the opening 158. The opening 158 may be located within a distal surface of the removable protective cap 148 facing the skin of the user. The removable protective cap 148 may further be mechanically interlocked with the sterility cap 116 within the opening 158.

Further, the sterility cap 116 may be engaged with the removable protective cap 148, such that, when the removable protective cap 148 may be removed from the housing 146, the sterility cap 116 may be removed from preassembled functional module 110. For example, the sterility cap 116 may be mechanically interlocked with the removable protective cap 148. Thus, the mechanical interlock between the sterility cap 116 and the removable protective cap 148 may be accomplished by corresponding interlocking elements 159 of the sterility cap 116 and the removable protective cap 148, respectively, such as by one or more notches, hooks, protrusions, dents or the like, as the skilled person will recognize.

In order to prepare the medical system 144 for use, the user may remove the removable protective cap 148 from the housing 146, thereby removing the sterility cap 116 from the insertion component 114. The insertion component 114 may, thus, be revealed and the medical system 144 may be ready for use. Thereafter, the user may place a base part 138 of the housing 146 onto the skin site and actuate the insertion, as will be outlined in further detail below. As an example, the base part 138 may be or may comprise a distal edge or front rim 139, such as a circular front rim, for placement onto the skin site during insertion.

The sterility cap 116 may be arranged such that the sterility cap 116 may have at least one side in common with the removable protective cap 148. For example, the sterility testing access 140 may be located at the side which is in common with the removable protective cap 148. The side in common with the removable protective cap 148 specifically may be a distal side, facing the skin of the user.

The medical system 144 may further comprise at least one driving actuator 160. The driving actuator 160 may be configured for driving the insertion component 114 into a body tissue of the user. For example, the driving actuator 160 may engage with the plunger 136. The driving actuator 160 may be movable with respect to the base part 138 of the preassembled functional module 110. The driving actuator 160 may be movable in a forward direction, specifically in a direction towards the skin of a user. Thus, the driving actuator 160 may be configured for driving the insertion component 114 into the body tissue of the user. Further, the driving actuator 160 may be movable in a backward direction, specifically in a direction opposite to the forward direction, specifically after insertion. Thus, the driving actuator 160 may retract the insertion component 114 after the analyte sensor 112 has been inserted into the body tissue of the user, whereas the analyte sensor 112, at least with its inserted part, remains within the body tissue. The movement of the driving actuator 160, as an example, may be driven manually, such as by the user exerting a force onto the driving actuator 160. The movement into the backward direction specifically may be driven by a return spring 167.

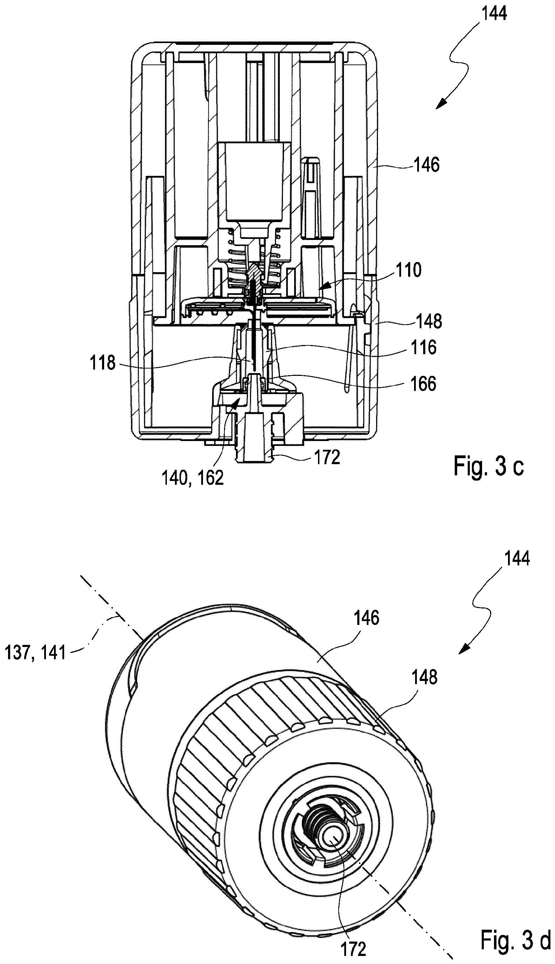

In Figures 3a-d, a second exemplary embodiment of the medical system 144 is shown various views and states. The medical system 144 may, apart from the design of the sterility testing access 140, be widely identical to the first embodiment shown in Figure 2 above, so reference may be made to the description of Figure 2 above. Figures 3a and 3c show cross-sectional views of the second embodiment of the medical system 144, and Figures 3b and 3d show perspective views. Further, Figures 3a and 3b show the medical system 144 in a state in which a removable stopper 170 is attached to the sterility cap 116, which may be the normal state in which the medical system 144 may be stored, sold or used for insertion. Figures 3c and 3d show a second state of the medical system 144 in which the removable stopper 170 is removed and replaced by a fluidic adapter 172, for the purpose of sterility testing.

As outlined above, the medical system 144 in Figures 3a-d may widely correspond to the medical system 144 shown in Figure 2, apart from the design of the sterility testing access 140. Thus, the sterility testing access 140 in the second embodiment shown in Figures 3a-d comprises a multiple-step sealing 162 which will be explained in further detail below.

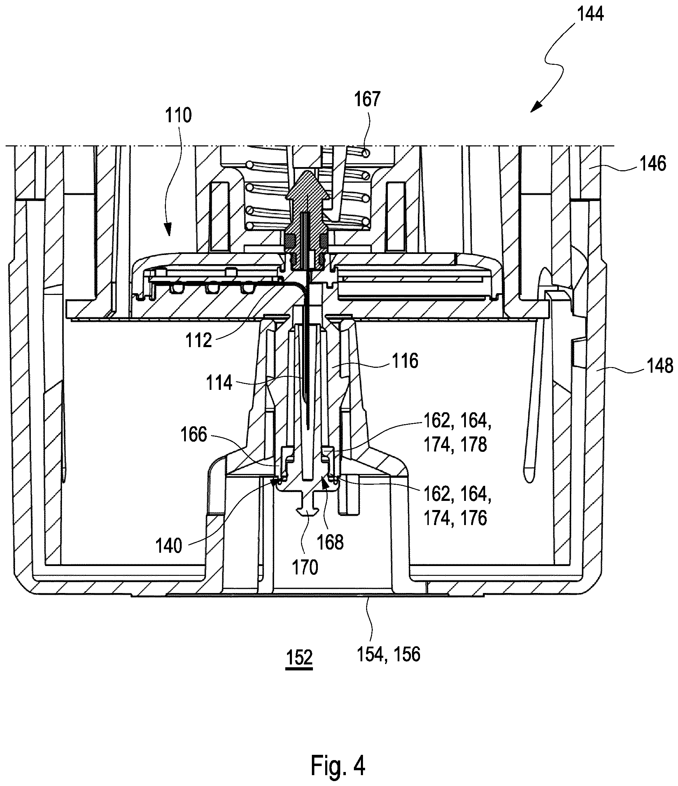

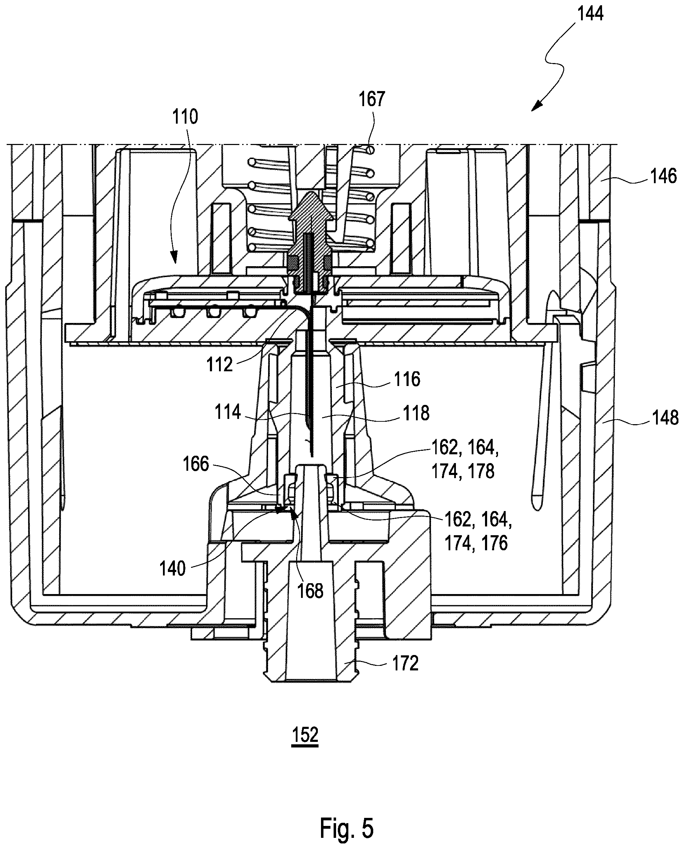

Thus, in Figure 4, an enlarged partial view of the medical system 144 of Figures 3a and 3b is shown in a cross-sectional view. In Figure 5, an enlarged view of the medical system 144 of Figures 3c and 3d is shown in a cross-sectional view. In the following, the multiple-step sealing 162 will be explained with respect to these Figures 3a-d, 4 and 5 in conjunction.

The multiple-step sealing 162, as visible e.g. in Figures 4 and 5, may comprise a plurality of circumferential sealing elements 164, such as two circumferential sealing elements 164. The circumferential sealing elements 164 may be disposed concentrically, specifically with respect to an axis of extension 141 of the sterility cap 116. The axis of extension 141 of the sterility cap 116 may be identical to the axis of rotational symmetry 137, such as the cylindrical axis. The cylindrical symmetry of the medical system 144 is shown in Figure 3b, wherein the medical system 144 is shown in a perspective view.

Further, the sterility cap 116 may comprise a tubular sidewall 166. The tubular sidewall 166 may surround a sterility testing access opening 168 located at a distal end of the sterility cap 116. The sterility testing access opening 168 may be closed by the removable stopper 170. In Figures 3a-b and 4, the removable stopper 170 may seal the sterility testing access opening 168. The removable stopper 170 may be removed prior to sterility testing of the medical system 144. Thus, for sterility testing, the removable stopper 170 may be replaced by a fluidic adapter 172 which may be connected to the multiple-step sealing 162. This situation is shown in Figures 3c-d and 5. The fluidic adapter 172 may be attached to the sterility testing access 140. The fluidic adapter 172 may be configured for inserting and removing a rinsing liquid to the interior space 118 of the sterility cap 116, such as for flushing the interior space 118 with the rinsing liquid. A detailed view of the multiple-step sealing 162 is shown in Figures 4 and 5.

The multiple-step sealing 162 may be configured for sealing the sterility testing access 140 of the medical system 144. As outlined above, the multiple-step sealing 162 may comprise a plurality of circumferential sealing elements 164, specifically disposed within the sterility testing access opening 168. For example, the multiple-step sealing 162 may be a two-step sealing 174. The two-step sealing 174 may comprise a first-step sealing 176 and a second-step sealing 178. The two-step sealing 174 may also comprise circumferential sealing elements 164 which may be disposed concentrically, specifically with respect to an axis of extension 141 of the sterility cap 116.

Further, the two circumferential sealing elements 164 may comprise the tubular sidewall 166 surrounding the sterility testing access opening 168. The sterility testing access 140 of the sterility cap 116 may be disposed within the sterility testing access opening 168. The sterility testing access opening 168 may be closed by the removable stopper 170. Thus, the two-step sealing 174 may be configured for receiving the removable stopper 170 at a first-step sealing 176 of the multiple-step sealing 162. The removable stopper 170 may seal the sterility testing access 140 specifically at the first-step sealing 176.

In case the sterility of the medical system 144 shall be tested, the sterility testing access 140 may be accessible from the outer side 152 of the medical system 144 through the removable protective cap 148. For this purpose, the removable protective cap 148 may remain connected to the housing 146 of the medical system 144 during sterility testing. A removal of the removable seal 154 comprised by the removable protective cap 148 may reveal the sterility testing access 140. Thus, the removable stopper 170 sealing the sterility testing access 140 may be removed from the outer side 152 of the medical system 144.

In Figure 5, an exemplary embodiment of the fluidic adapter 172 attached to the multiple-step sealing 162 of the medical system 144 is shown. Therein, the removable stopper 170 is removed from the sterility testing access 140. The stopper 170 may be removed prior to sterility testing. After the removal of the removable stopper 170, the fluidic adapter 172 may be attached to the sterility testing access 140. The fluidic adapter 172 may be configured for inserting the rinsing liquid into the interior space 118 of the sterility cap 116. The fluidic adapter 172 may be in contact with the second-step sealing 178 of the multiple-step sealing 162. Specifically, the second-step sealing 178 may have a smaller equivalent diameter than the first-step sealing 176. Thus, the fluidic adapter 172 may not contact the first-step sealing 176. The risk of a carryover of a contamination present on the first-step sealing 176 into the interior space 118 may be minimized.

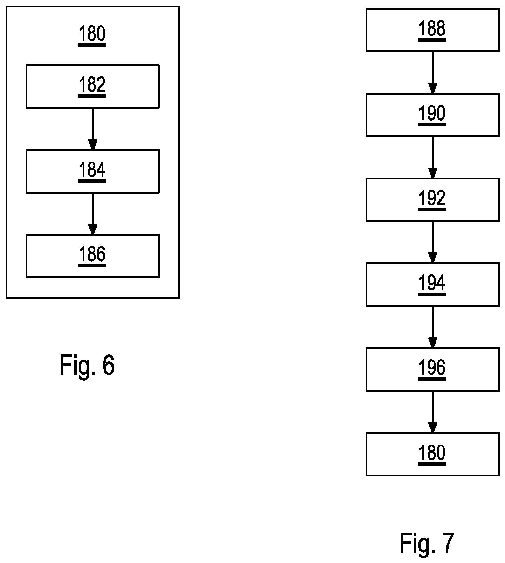

In Figure 6, an exemplary embodiment of a method of sterility testing of the medical system 144 is shown in a flowchart. The method of sterility testing comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The method of sterility testing may comprise additional method steps that are not listed.

The medical system 144 specifically may be embodied according to any one of the embodiments disclosed above. At least, however, the medical system 144 comprises the preassembled functional module 110, wherein the preassembled functional module 110 comprises an analyte sensor 112 for detecting at least one analyte in a body fluid of a user. Further, the preassembled functional module 110 comprises at least one electronics unit 120 electrically connected to the analyte sensor 112 and an insertion component 114 for inserting the analyte sensor 112 into a body tissue of the user. The preassembled functional module 110 further comprises at least one sterility cap 116 at least partially surrounding the insertion component 114 and optionally at least a part of the analyte sensor 112.

The method of sterility testing 180 of the medical system 144 comprise the following steps:
- (denoted with reference number 182) inserting the rinsing liquid into an interior space 118 of the sterility cap 116 (step A);
- optionally: (denoted with reference number 184) removing the rinsing liquid from the interior space 118 of the sterility cap 116; and
- (denoted with reference number 186) microbial testing of the rinsing liquid (step B).

As outlined above, step 184 is an optional step. Thus, the microbial testing in step 186 may also be performed while at least part of the rinsing liquid still is in the interior space 118, e.g. while a reservoir of the rinsing liquid is still attached to the sterility cap 116.

In step A), the rinsing liquid may be applied to the interior space 118 through the sterility testing access 140 of the sterility cap 116. Thus, step A) may further comprise attaching the fluidic adapter 172 to the sterility testing access 140. For example, the fluidic adapter 172 may be a sterile cannula which is configured for piercing the septum 142 comprised by the sterility testing access 140. The rinsing liquid may be inserted into and removed from the interior space 118 through the cannula. As another example, the fluidic adapter 172 may be embodied by the fluidic adapter 172 shown in Figure 5. Thus, the fluidic adapter 172 may be connected to the multiple-step sealing 162 of the sterility testing access 140. The fluidic adapter 172 may provide a fluidic connection from the outer side 152 of the medical system 144 to the interior space 118 of the sterility cap 116.

Alternatively, however, the embodiment of the medical system 144 of Figure 2 may be used. In this embodiment, step A) may be performed by inserting and removing the rinsing liquid trough the septum 142. As an example, the septum 142 may be pierced by a needle or cannula of a syringe by which the rinsing liquid is inserted into the interior space 118 and by which the rinsing liquid may also be removed from the interior space 118.

The rinsing liquid may specifically be applied to the interior space 118 without contacting the housing 146 of the medical system 144. The rinsing liquid may further be inserted to the interior space 118 in such a way that a possible contamination of the sterile parts, such as the insertion component 114 and part of the analyte sensor 112, may be incorporated into the rinsing liquid.

Step B) of the method of sterility testing may comprise incubating the rinsing liquid. Thus, a possible contamination incorporated into the rinsing liquid may be determined. After incubating the rinsing liquid, the sterility testing 180 may comprise the result, whether the insertion component 114 and the analyte sensor 112 were sterile or not.

As outlined above, step A) of the method of sterility testing may comprise attaching the fluidic adapter 172 to the sterility testing access 140 and inserting at least a part of the rinsing liquid into the interior space 118 of the sterility cap 116. In particular, at least a part of the rinsing liquid may remain outside the interior space 118 of the sterility cap 116, e.g. in a reservoir, but may fluidically be in contact with the rinsing liquid inside the interior space 118. In this state, step B) of the method may be performed. Thus, the rinsing liquid may be incubated with the fluidic adapter 172 attached to the sterility testing access 140, wherein the rinsing liquid outside the interior space 118 may be in fluidic contact with the sterile parts inside the interior space 118 of the sterility cap 116.

Figure 7 shows a flowchart of an exemplary embodiment of a method of providing the medical system 144. The medical system 144 specifically may be embodied according to any one of the embodiments disclosed above. Thus, for optional details, reference may be made to the description of these embodiments. The method of providing the medical system 144 comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The method of providing the medical system 144 may comprise additional method steps that are not listed.

The method of providing the medical system 144 comprises the following steps:
I. (denoted with reference number 188) assembling a part 195 of the preassembled functional module 110, the part 195 of the preassembled functional module 110 comprising the analyte sensor 112 and the insertion component 114 as well as the sterility cap 116 at least partially surrounding the insertion component 114;
II. (denoted with reference number 190) sterilizing the part 195 of the preassembled functional module 110, specifically by using one or more of e-beam or gamma sterilization;
III. (denoted with reference number 192) assembling the preassembled functional module 110, the assembling comprising electrically connecting the electronics unit 120 to the analyte sensor 112; and
IV. (denoted with reference number 194) assembling the medical system 144, the assembling comprising receiving the preassembled functional module 110 in the housing 146 and connecting the removable protective cap 148 to the housing 146.

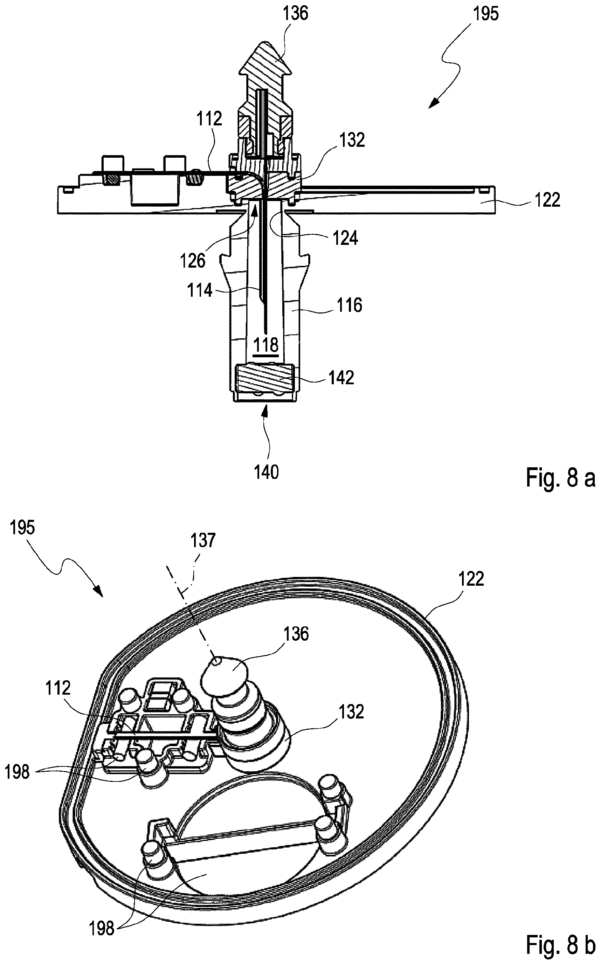

Step I. of the method of providing the medical system 144 may refer to an assembly of a part of the medical system 144 which comprises at least some of the components to be sterilized. The part 195 assembled in step I. may form a subgroup of the preassembled functional module 110. In Figures 8a and 8b, an exemplary embodiment of this part 195 or subgroup of the preassembled functional module 110 is shown in a cross-sectional view (Figure 8a) and in a perspective view (Figure 8b). Therein, the preassembled functional module 110 corresponds to the embodiment of Figures 1a-b and 2.

The part 195 or subgroup, as shown in Figures 8a-b, comprises at least some of the sterile parts of the medical system 144. The sterile parts may comprise at least the analyte sensor 112, the insertion component 114 and/or the sterility cap 116. Further, the subgroup may comprise the sensor fixation element 132 and/or the base plate 122. As can be seen in Figure 8b, the base plate 122, as an example, may comprise one or more structural elements 198, such as for mounting the electronic unit 120, for electrically contacting the analyte sensor 112, for receiving an electrical energy storage device or the like. The subgroup may be assembled under clean conditions, such as in a clean room facility or the like.

After performing step I., i.e. after assembling part 195, part 195 may be subjected to the sterilization in step II. For this purpose, as an example, the subgroup may be transferred to a sterilization unit, such as an X-ray sterilizer, a gamma sterilizer, an e-beam sterilizer or the like. At this stage, preferably, no components which are prone to electronic damages by the sterilization process are present in the subgroup, such as the electronics unit 120.

After performing step II., the part 195, also referred to as the subgroup, is a sterilized part or sterilized subgroup. Thus, specifically, the interior space 118 is in a sterilized condition, including the parts received therein, such as the insertion component 114 and the part of the analyte sensor 112 received therein.

The sterilized part 195 may then undergo further processing, wherein, however, the sterilized state of at least the interior space 118 and the parts received therein remains unchanged.

Thus, in step III., the electronics unit 120 may be attached to the part 195. Further, the cover element 134 may be mounted, thereby closing the preassembled functional module 110. Further, the adhesive plaster 123 may be applied to the base plate 122.

In step IV., the preassembled functional module 110 may be combined with other components of the medical system 144. Thus, the preassembled functional module 110 may be inserted into the housing 146. Further, the removable protective cap 148 may be attached to the housing 146, and the removable seal 154 may be applied.

Thus, the method of providing the medical system 144 may comprise an assembly process wherein sterile and non-sterile part may be processed.

The method of providing the medical system 144 may further comprise:
V. (denoted by reference number 196) storing the medical system 144.

After storing the medical system 144, the method of providing the medical system 144 may further comprise:
VI. (denoted by reference number 180) sterility testing of the medical system 144 by using the method of sterility testing of a medical system 144.

The sterility of the medical system 144 may be tested by performing step VI. Step VI. may be performed on few selected medical systems 144. The medical systems 144 subjected to sterility testing 180 may be selected on a random basis, specifically in such a way that the sterility testing 180 of the few selected medical systems 144 may allow for verifying the sterility of all assembled medical systems 144. For example, a percentage of sterility tested medical systems 144 to assembled medical systems 144 may be less than 1%, e.g. 0.1 % or even less.

### List of reference numbers

- 110: preassembled functional module
- 112: analyte sensor
- 114: insertion component
- 116: sterility cap
- 118: interior space
- 120: electronics unit
- 122: base plate
- 123: adhesive plaster
- 124: predetermined breaking point
- 126: through hole
- 128: upper side
- 130: lower side
- 132: sensor fixation element
- 134: cover element
- 136: plunger
- 137: symmetry axis
- 138: base part
- 139: front rim
- 140: sterility testing access
- 141: axis of extension
- 142: septum
- 144: medical system
- 146: housing
- 147: rim of the removable protective cap
- 148: removable protective cap
- 149: rim of the housing
- 150: funnel-shaped depression
- 152: outer side
- 154: removable seal
- 156: removable liner
- 158: opening
- 159: interlocking element
- 160: driving actuator
- 162: multiple-step sealing
- 164: circumferential sealing elements
- 166: tubular sidewall
- 167: return spring
- 168: sterility testing access opening
- 170: removable stopper
- 172: fluidic adapter
- 174: two-step sealing
- 176: first-step sealing
- 178: second-step sealing
- 180: sterility testing
- 182: inserting a rinsing liquid
- 184: removing a rinsing liquid
- 186: microbial testing of a rinsing liquid
- 188: assembling a part of a preassembled functional module
- 190: sterilizing a part of a preassembled functional module
- 192: assembling a preassembled functional module
- 194: assembling a medical system
- 195: part of the preassembled functional module
- 196: storing a medical system
- 198: structural elements

## Claims

1. A medical system (144) comprising:
i) a housing (146);
ii) a preassembled functional module (110) received in the housing (146), the preassembled functional module (110) comprising
a. an analyte sensor (112) for detecting at least one analyte in a body fluid of a user;
b. an electronics unit (120) electrically connected to the analyte sensor (112); and
c. an insertion component (114) for inserting the analyte sensor (112) into a body tissue of the user; and
iii) at least one removable protective cap (148) connected to the housing (146), covering the preassembled functional module (110),
wherein the preassembled functional module (110) further comprises at least one sterility cap (116), which at least partially surrounds the insertion component (114), wherein the at least one sterility cap (116) is at least partially surrounded by the protective cap (148), wherein the sterility cap (116) comprises at least one sterility testing access (140), the sterility testing access (140) comprising at least one of a septum (142) and a multiple-step sealing (162).

2. The medical system (144) according to claim 1, wherein the multiple-step sealing (162) comprises a plurality of circumferential sealing elements (164) having differing equivalent diameters, the circumferential sealing elements (164) being disposed within an interior space (118) of the sterility cap (116).

3. The medical system (144) according to any one of claims 1 or 2, wherein the sterility cap (116) comprises a tubular sidewall (166) surrounding a sterility testing access opening (168), wherein the sterility testing access opening (168) is closed by at least one of the septum (142) and a removable stopper (170).

4. The medical system (144) according to any one of claims 1-3, wherein the sterility cap (116) is accessible from an outer side (152) of the medical system (144), wherein an access to the sterility cap (116) is sealed by a removable seal (152).

5. The medical system (144) according to any one of claims 1-4, wherein the sterility cap (116) is engaged with the removable protective cap (148), such that, when the protective cap (148) is removed from the housing (146), the sterility cap (116) is removed from the preassembled functional module (110).

6. The medical system (144) according to any one of claims 1-5, wherein the sterility cap (116) has at least one side in common with the protective cap (148).

7. The medical system (144) according to any one of claims 1-6, wherein the preassembled functional module (110) further comprises at least one base plate (122), wherein the sterility cap (116) is connected to the base plate (122).

8. The medical system (144) according to any one of claims 1-7, wherein the preassembled functional module (110) further comprises at least one cover element (134), wherein the cover element (134) covers the electronics unit (120) and at least part of the analyte sensor (112).

9. The medical system (144) according to any one of claims 1-8, wherein the sterility cap (116) provides a sealed interior space (118), wherein the analyte sensor (112) and the insertion component (114) at least partially are located within the sealed interior space (118).

10. The medical system (144) according to any one of claims 1-9, wherein the medical system (144) further comprises at least one driving actuator (160) for driving the insertion component (114) into the body tissue of the user.

11. The medical system (144) according to any one of claims 1-10, wherein the preassembled functional module (110) further comprises at least one electrical energy storage device.

12. A method of sterility testing of a medical system (144), the medical system (144) comprising a preassembled functional module (110), the preassembled functional module (110) comprising an analyte sensor (112) for detecting at least one analyte in a body fluid of a user, and an electronics unit (120) electrically connected to the analyte sensor (112) and an insertion component (114) for inserting the analyte sensor (112) into a body tissue of the user, wherein the preassembled functional module (110) further comprises at least one sterility cap (116) at least partially surrounding the insertion component (114) and optionally at least a part of the analyte sensor (112), wherein the method comprises:
A) inserting a rinsing liquid into an interior space (118) of the sterility cap (116); and
B) microbial testing of the rinsing liquid.

13. The method according to claim 12, wherein the sterility cap (116) has at least one sterility testing access (140), wherein step A) comprises attaching at least one fluidic adapter (172) to the sterility testing access (140), wherein the sterility testing access (140) comprises at least one multiple-step sealing (162), wherein step A) comprises removing at least one stopper from the sterility testing access (140), the stopper being in contact with a first-step sealing (176) of the multiple-step sealing (162), step A) further comprising attaching the fluidic adapter (172) to the sterility testing access (140), wherein the fluidic adapter (172) is in contact with a second-step sealing (178) of the multiple-step sealing (162), wherein the first-step sealing (176) has a larger equivalent diameter than the second-step sealing (178).

14. A method of providing the medical system (144) according to any one of claims 1-11, the method comprising:
I. assembling a part (195) of the preassembled functional module (110), the part (195) of the preassembled functional module (110) comprising the analyte sensor (112) and the insertion component (114) as well as the sterility cap (116) at least partially surrounding the insertion component (114);
II. sterilizing the part (195) of the preassembled functional module (110);
III. assembling the preassembled functional module (110), the assembling comprising electrically connecting the electronics unit (120) to the analyte sensor (112); and
IV. assembling the medical system (144), the assembling comprising receiving the preassembled functional module (110) in the housing (146) and connecting the removable protective cap (148) to the housing (146).

15. The method according to claim 14, the method further comprising:
V. storing the medical system (144).

16. The method according to any one of claims 14-15, the method further comprising:
VI. sterility testing of the medical system (144) by using the method of sterility testing of a medical system (144) according to any one of claims 12-13.

## Patentansprüche

1. Medizinisches System (144), umfassend:
i) ein Gehäuse (146);
ii) ein vormontiertes Funktionsmodul (110), das in dem Gehäuse (146) aufgenommen ist, wobei das vormontierte Funktionsmodul (110) Folgendes umfasst
a. einen Analytsensor (112) zum Nachweisen mindestens eines Analyten in einer Körperflüssigkeit eines Benutzers;
b. eine Elektronikeinheit (120), die elektrisch mit dem Analytsensor (112) verbunden ist; und
c. eine Einführkomponente (114) zum Einführen des Analytsensors (112) in ein Körpergewebe des Benutzers; und
iii) mindestens eine entfernbare Schutzkappe (148), die mit dem Gehäuse (146) verbunden ist und das vormontierte Funktionsmodul (110) abdeckt,
wobei das vormontierte Funktionsmodul (110) ferner mindestens eine Sterilitätskappe (116) umfasst, die die Einführkomponente (114) mindestens teilweise umgibt, wobei die mindestens eine Sterilitätskappe (116) mindestens teilweise von der Schutzkappe (148) umgeben ist, wobei die Sterilitätskappe (116) mindestens einen Sterilitätstestzugang (140) umfasst, wobei der Sterilitätstestzugang (140) mindestens eines von einem Septum (142) und einer mehrstufigen Dichtung (162) umfasst.

2. Medizinisches System (144) nach Anspruch 1, wobei die mehrstufige Dichtung (162) eine Vielzahl von umlaufenden Dichtungselementen (164) mit unterschiedlichen Äquivalentdurchmessern umfasst, wobei die umlaufenden Dichtungselemente (164) in einem Innenraum (118) der Sterilitätskappe (116) angeordnet sind.

3. Medizinisches System (144) nach einem der Ansprüche 1 oder 2, wobei die Sterilitätskappe (116) eine röhrenförmige Seitenwand (166) umfasst, die eine Sterilitätstestzugangsöffnung (168) umgibt, wobei die Sterilitätstestzugangsöffnung (168) von mindestens einem von dem Septum (142) und einem entfernbaren Stopfen (170) verschlossen wird.

4. Medizinisches System (144) nach einem der Ansprüche 1-3, wobei die Sterilitätskappe (116) von einer Außenseite (152) des medizinischen Systems (144) zugänglich ist, wobei ein Zugang zu der Sterilitätskappe (116) von einer entfernbaren Dichtung (152) abgedichtet wird.

5. Medizinisches System (144) nach einem der Ansprüche 1-4, wobei die Sterilitätskappe (116) mit der entfernbaren Schutzkappe (148) in Eingriff steht, so dass, wenn die Schutzkappe (148) von dem Gehäuse (146) entfernt wird, die Sterilitätskappe (116) von dem vormontierten Funktionsmodul (110) entfernt wird.

6. Medizinisches System (144) nach einem der Ansprüche 1-5, wobei die Sterilitätskappe (116) mindestens eine gemeinsame Seite mit der Schutzkappe (148) aufweist.

7. Medizinisches System (144) nach einem der Ansprüche 1-6, wobei das vormontierte Funktionsmodul (110) ferner mindestens eine Grundplatte (122) umfasst, wobei die Sterilitätskappe (116) mit der Grundplatte (122) verbunden ist.

8. Medizinisches System (144) nach einem der Ansprüche 1-7, wobei das vormontierte Funktionsmodul (110) ferner mindestens ein Abdeckelement (134) umfasst, wobei das Abdeckelement (134) die Elektronikeinheit (120) und mindestens einen Teil des Analytsensors (112) abdeckt.

9. Medizinisches System (144) nach einem der Ansprüche 1-8, wobei die Sterilitätskappe (116) einen abgedichteten Innenraum (118) bereitstellt, wobei sich der Analytsensor (112) und die Einführkomponente (114) mindestens teilweise in dem abgedichteten Innenraum (118) befinden.

10. Medizinisches System (144) nach einem der Ansprüche 1-9, wobei das medizinische System (144) ferner mindestens einen Antriebsaktuator (160) zum Antreiben der Einführkomponente (114) in das Körpergewebe des Benutzers umfasst.

11. Medizinisches System (144) nach einem der Ansprüche 1-10, wobei das vormontierte Funktionsmodul (110) ferner mindestens eine Speichervorrichtung für elektrische Energie umfasst.

12. Verfahren zum Testen der Sterilität eines medizinischen Systems (144), wobei das medizinische System (144) ein vormontiertes Funktionsmodul (110) umfasst, wobei das vormontierte Funktionsmodul (110) einen Analytsensor (112) zum Nachweisen mindestens eines Analyten in einer Körperflüssigkeit eines Benutzers und eine Elektronikeinheit (120), die elektrisch mit dem Analytsensor (112) verbunden ist, und eine Einführkomponente (114) zum Einführen des Analytsensors (112) in ein Körpergewebe des Benutzers umfasst, wobei das vormontierte Funktionsmodul (110) ferner mindestens eine Sterilitätskappe (116) umfasst, die die Einführkomponente (114) mindestens teilweise und gegebenenfalls mindestens einen Teil des Analytsensors (112) umgibt, wobei das Verfahren Folgendes umfasst:
A) Einführen einer Spülflüssigkeit in einen Innenraum (118) der Sterilitätskappe (116);
und
B) mikrobielles Testen der Spülflüssigkeit.

13. Verfahren nach Anspruch 12, wobei die Sterilitätskappe (116) mindestens einen Sterilitätstestzugang (140) aufweist, wobei Schritt A) das Anbringen mindestens eines Fluidikadapters (172) an den Sterilitätstestzugang (140) umfasst, wobei der Sterilitätstestzugang (140) mindestens eine mehrstufige Dichtung (162) umfasst, wobei Schritt A) das Entfernen mindestens eines Stopfens von dem Sterilitätstestzugang (140) umfasst, wobei der Stopfen mit einer Dichtung der ersten Stufe (176) der mehrstufigen Dichtung (162) in Kontakt ist, wobei Schritt A) ferner das Anbringen des Fluidikadapters (172) an den Sterilitätstestzugang (140) umfasst, wobei der Fluidikadapter (172) mit einer Dichtung der zweiten Stufe (178) der mehrstufigen Dichtung (162) in Kontakt ist, wobei die Dichtung der ersten Stufe (176) einen größeren Äquivalentdurchmesser aufweist als die Dichtung der zweiten Stufe (178).

14. Verfahren zum Bereitstellen des medizinischen Systems (144) nach einem der Ansprüche 1-11, wobei das Verfahren Folgendes umfasst:
I. Montieren eines Teils (195) des vormontierten Funktionsmoduls (110), wobei das Teil (195) des vormontierten Funktionsmoduls (110) den Analytsensor (112) und die Einführkomponente (114) sowie die Sterilitätskappe (116), die die Einführkomponente (114) mindestens teilweise umgibt, umfasst;
II. Sterilisieren des Teils (195) des vormontierten Funktionsmoduls (110);
III. Montieren des vormontierten Funktionsmoduls (110), wobei das Montieren das elektrische Verbinden der Elektronikeinheit (120) mit dem Analytsensor (112) umfasst; und
IV. Montieren des medizinischen Systems (144), wobei das Montieren das Aufnehmen des vormontierten Funktionsmoduls (110) in dem Gehäuse (146) und das Verbinden der entfernbaren Schutzkappe (148) mit dem Gehäuse (146) umfasst.

15. Verfahren nach Anspruch 14, wobei das Verfahren ferner Folgendes umfasst:
V. Lagern des medizinischen Systems (144).

16. Verfahren nach einem der Ansprüche 14-15, wobei das Verfahren ferner Folgendes umfasst:
VI. Testen der Sterilität des medizinischen Systems (144) unter Anwendung des Verfahrens zum Testen der Sterilität eines medizinischen Systems (144) nach einem der Ansprüche 12-13.

## Revendications

1. Système médical (144) comprenant :
i) un boîtier (146) ;
ii) un module fonctionnel préassemblé (110) logé dans le boîtier (146), le module fonctionnel préassemblé (110) comprenant
a. un capteur d'analyte (112) pour détecter au moins un analyte dans un fluide corporel d'un utilisateur ;
b. une unité électronique (120) connectée électriquement au capteur d'analyte (112) ; et
c. un composant d'insertion (114) pour insérer le capteur d'analyte (112) dans un tissu corporel de l'utilisateur ; et
iii) au moins un capuchon de protection amovible (148) relié au boîtier (146), recouvrant le module fonctionnel préassemblé (110),
dans lequel le module fonctionnel préassemblé (110) comprend en outre au moins un capuchon de stérilité (116), qui entoure au moins partiellement le composant d'insertion (114), dans lequel l'au moins un capuchon de stérilité (116) est au moins partiellement entouré par le capuchon de protection (148), dans lequel le capuchon de stérilité (116) comprend au moins un accès de test de stérilité (140), l'accès de test de stérilité (140) comprenant au moins un parmi un septum (142) et un dispositif d'étanchéité multi-étape (162).

2. Système médical (144) selon la revendication 1, dans lequel le dispositif d'étanchéité multi-étape (162) comprend une pluralité d'éléments d'étanchéité circulaires (164) ayant des diamètres équivalents différents, les éléments d'étanchéité circulaires (164) étant disposés au sein d'un espace intérieur (118) du capuchon de stérilité (116).

3. Système médical (144) selon l'une quelconque des revendications 1 ou 2, dans lequel le capuchon de stérilité (116) comprend une paroi latérale tubulaire (166) entourant une ouverture d'accès de test de stérilité (168), dans lequel l'ouverture d'accès de test de stérilité (168) est fermée par au moins un parmi le septum (142) et un bouchon amovible (170).

4. Système médical (144) selon l'une quelconque des revendications 1 à 3, dans lequel le capuchon de stérilité (116) est accessible depuis un côté extérieur (152) du système médical (144), dans lequel un accès au capuchon de stérilité (116) est rendu étanche par un joint d'étanchéité amovible (152).

5. Système médical (144) selon l'une quelconque des revendications 1 à 4, dans lequel le capuchon de stérilité (116) est en prise avec le capuchon de protection amovible (148), de telle sorte que, lorsque le capuchon de protection (148) est retiré du boîtier (146), le capuchon de stérilité (116) est retiré du module fonctionnel préassemblé (110).

6. Système médical (144) selon l'une quelconque des revendications 1 à 5, dans lequel le capuchon de stérilité (116) a au moins un côté en commun avec le capuchon de protection (148).

7. Système médical (144) selon l'une quelconque des revendications 1 à 6, dans lequel le module fonctionnel préassemblé (110) comprend en outre au moins une plaque de base (122), dans lequel le capuchon de stérilité (116) est relié à la plaque de base (122).

8. Système médical (144) selon l'une quelconque des revendications 1 à 7, dans lequel le module fonctionnel préassemblé (110) comprend en outre au moins un élément de couvercle (134), dans lequel l'élément de couvercle (134) recouvre l'unité électronique (120) et au moins une partie du capteur d'analyte (112).

9. Système médical (144) selon l'une quelconque des revendications 1 à 8, dans lequel le capuchon de stérilité (116) fournit un espace intérieur rendu étanche (118), dans lequel le capteur d'analyte (112) et le composant d'insertion (114) sont au moins partiellement situés au sein de l'espace intérieur rendu étanche (118).

10. Système médical (144) selon l'une quelconque des revendications 1 à 9, dans lequel le système médical (144) comprend en outre au moins un actionneur d'entraînement (160) pour entraîner le composant d'insertion (114) dans le tissu corporel de l'utilisateur.

11. Système médical (144) selon l'une quelconque des revendications 1 à 10, dans lequel le module fonctionnel préassemblé (110) comprend en outre au moins un dispositif de stockage d'énergie électrique.

12. Procédé de test de stérilité d'un système médical (144), le système médical (144) comprenant un module fonctionnel préassemblé (110), le module fonctionnel préassemblé (110) comprenant un capteur d'analyte (112) pour détecter au moins un analyte dans un fluide corporel d'un utilisateur, et une unité électronique (120) connectée électriquement au capteur d'analyte (112) et un composant d'insertion (114) pour insérer le capteur d'analyte (112) dans un tissu corporel de l'utilisateur, dans lequel le module fonctionnel préassemblé (110) comprend en outre au moins un capuchon de stérilité (116) entourant au moins partiellement le composant d'insertion (114) et éventuellement au moins une partie du capteur d'analyte (112), dans lequel le procédé comprend :
A) l'insertion d'un liquide de rinçage dans un espace intérieur (118) du capuchon de stérilité (116) ; et
B) le test microbien du liquide de rinçage.

13. Procédé selon la revendication 12, dans lequel le capuchon de stérilité (116) a au moins un accès de test de stérilité (140), dans lequel l'étape A) comprend la fixation d'au moins un adaptateur fluidique (172) à l'accès de test de stérilité (140), dans lequel l'accès de test de stérilité (140) comprend au moins un dispositif d'étanchéité multi-étape (162), dans lequel l'étape A) comprend le retrait d'au moins un bouchon de l'accès de test de stérilité (140), le bouchon étant en contact avec un dispositif d'étanchéité de première étape (176) du dispositif d'étanchéité multi-étape (162), l'étape A) comprenant en outre la fixation de l'adaptateur fluidique (172) à l'accès de test de stérilité (140), dans lequel l'adaptateur fluidique (172) est en contact avec un dispositif d'étanchéité de seconde étape (178) du dispositif d'étanchéité multi-étape (162), dans lequel le dispositif d'étanchéité de première étape (176) a un diamètre équivalent supérieur plus important que le dispositif d'étanchéité de seconde étape (178).

14. Procédé de fourniture du système médical (144) selon l'une quelconque des revendications 1 à 11, le procédé comprenant :
I. l'assemblage d'une partie (195) du module fonctionnel préassemblé (110), la partie (195) du module fonctionnel préassemblé (110) comprenant le capteur d'analyte (112) et le composant d'insertion (114) ainsi que le capuchon de stérilité (116) entourant au moins partiellement le composant d'insertion (114) ;
II. la stérilisation de la partie (195) du module fonctionnel préassemblé (110) ;
III. l'assemblage du module fonctionnel préassemblé (110), l'assemblage comprenant la connexion électrique de l'unité électronique (120) au capteur d'analyte (112) ; et
IV. l'assemblage du système médical (144), l'assemblage comprenant la réception du module fonctionnel préassemblé (110) dans le boîtier (146) et la liaison du capuchon de protection amovible (148) au boîtier (146).

15. Procédé selon la revendication 14, le procédé comprenant en outre :
V. le stockage du système médical (144).

16. Procédé selon l'une quelconque des revendications 14 à 15, le procédé comprenant en outre :
VI. le test de stérilité du système médical (144) en utilisant le procédé de test de stérilité d'un système médical (144) selon l'une quelconque des revendications 12 à 13.
